(19) Europäisches Patentamt
European Patent Office
Office européen des brevets

(11) **EP 2 026 852 B1**

(12) **EUROPEAN PATENT SPECIFICATION**

(45) Date of publication and mention
of the grant of the patent:
**12.01.2011 Bulletin 2011/02**

(51) Int Cl.:
*A61L 27/06* (2006.01)  *A61L 27/36* (2006.01)
*A61L 27/04* (2006.01)  *A61L 27/54* (2006.01)

(21) Application number: **07733748.3**

(22) Date of filing: **11.06.2007**

(86) International application number:
**PCT/GB2007/050327**

(87) International publication number:
**WO 2007/144667 (21.12.2007 Gazette 2007/51)**

(54) **METAL IMPLANTS**

METALLIMPLANTATE

IMPLANTS MÉTALLIQUES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **12.06.2006 GB 0611437**
**15.01.2007 GB 0700713**

(43) Date of publication of application:
**25.02.2009 Bulletin 2009/09**

(73) Proprietor: **Accentus Medical plc**
**3rd Floor, 11 Strand**
**London WC2N 5HR (GB)**

(72) Inventors:
• **PRENTICE, Thomas, Campbell**
**Andover Hampshire SP10 5JL (GB)**
• **PICKFORD, Martin, Edward, Lee**
**Southampton Hampshire SO31 6WB (GB)**
• **LEWIS, David, Richard**
**Abingdon Oxfordshire OX14 1XA (GB)**
• **TURNER, Andrew, Derek**
**Abingdon Oxfordshire OX14 1XR (GB)**

(74) Representative: **Mansfield, Peter Turquand et al**
**Coller IP Management Limited**
**Fugro House**
**Hithercroft Road**
**Wallingford, Oxfordshire OX10 9RB (GB)**

(56) References cited:
WO-A-03/089023    WO-A-03/094774
WO-A-2005/087982    WO-A-2006/058906

• **M. SHIRKHANZADEH ET AL.: "Bioactive delivery systems for the slow release of antibiotics: incorporation of Ag+ ions into micro-porous hydroxyapatite coatings." MATERIALS LETTERS, vol. 24, no. 1,2,3, 1995, pages 7-12, XP002482744**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

This invention relates to metal implants for use in surgical procedures where the implant is to be at least in partly in contact with bone, and in particular to the introduction of a biocidal material into such implants to suppress or control infection, and to a method of making such implants.

Various surgical procedures require the use of implants. For example cancerous bone may be removed, in prosthetic surgery, to be replaced by a metal implant. Such an implant may for example be of titanium alloy, which is very strong and relatively light. If part of the implant is to be movable relative to adjacent parts of the body then it is known to provide a smooth and polished surface on that part; and where part of the implant is to be embedded in bone it is known to provide a thermally sprayed coating containing hydroxyapatite (for example from US 4 746 532) to enhance growth of bone on to the implant.

A potential problem with any such implant is the risk of infection. As described in WO 2005/087982 a titanium metal implant can be treated to form a surface layer that is integral with the metal substrate and which incorporates a biocidal material. The method comprises anodising the implant in phosphoric acid at a voltage above 50 V for a period of at least 30 minutes, so as to generate a surface layer, and then performing ion exchange so as to incorporate ions of a biocidal metal into the surface layer. The surface is preferably polished prior to the anodising treatment. Anodising with the specified electrolyte and specified current density generates a hard surface coating of titania typically of thickness about 0.14 $\mu$m, but in which there are pits of diameter about 5 $\mu$m and depth about 0.4 $\mu$m which are filled with titanium oxide (or titanium phosphate) as a result of hydrolysis. Silver ions can then be incorporated, primarily in the material in these pits, to provide the required biocidal effect. However, this treatment is applicable primarily to implants comprising titanium.

WO 03/089023 discloses an implant comprising a metal substrate and a surface layer that is integral with the metal substrate, the layer incorporating a biocidal metal such as silver, deposited from a solution. Such an integral surface layer may be generated by an anodising process. This may form an adherent phosphate layer that may be modified to form a hydroxyapatite layer, which can stimulate bone growth. WO 2006/058906 discloses prosthetic devices formed by a substrate of any type of material provided with a coating of a glass, glass-ceramic or a ceramic material which are immersed in a silver-containing aqueous solution. WO 03/94774 discloses metallic implants having an oxide coating comprising calcium phosphate, which may be hydroxyapatite; the coating may comprise silver particles as an antimicrobial agent. M. Shirkhanzadeh, Materials Letters, vol. 24, 1995, pages 7-12, discloses hydroxyapatite coatings on metallic implants containing silver ions.

According to the present invention there is provided an implant for use at least partly in contact with bone, the implant comprising a metal structure, wherein the surface of the metal structure has an anodised hard oxide surface in which are small pits of ion absorbent material, wherein there is a ceramic coating containing hydroxyapatite deposited onto the anodised oxide at at least part of the surface of the metal structure; wherein silver ions which can gradually leach out into body fluids after implantation are contained within the ceramic coating or the anodised surface layer or both.

The use of hydroxyapatite coatings is recognized as improving bone ingrowth onto the implant. Silver is a biocidal material. The presence of silver in the coating appears to suppress collagenous in-growth while not inhibiting bone in-growth. The part of the implant which is to be in contact with bone preferably has a rough surface, which also enhances bonding to bone.

The present invention also provides a method of making an implant for use at least partly in contact with bone, the implant comprising a metal structure, the method comprising the steps of depositing onto at least part of the surface of the metal structure a ceramic coating containing hydroxyapatite by thermal spraying using a plasma spray system, and incorporating silver ions into the ceramic coating which can gradually leach out into body fluids after implantation.

Silver is suitable as the biocidal material because it is not particularly soluble in body fluids owing to the presence of chloride ions and the low solubility of silver chloride. The ceramic coating does not incorporate ions of other elements such as copper, tin, antimony, lead, bismuth, zinc or silicon. The silver ions are incorporated into the coating by ion exchange, and the coating is not subsequently fired, so the silver ions can gradually leach out into body fluids after implantation.

Preferably the part of the surface which is to be in contact with bone is first subjected to a roughening treatment, before being coated with the ceramic coating.

The ceramic coating is typically white. Preferably the silver ions are present in a form which neither alters the colour of the ceramic coating, nor changes its colour over time or on exposure to light. For example the hydroxyapatite may contain $Ag_2HPO_4$, which is white. The ions of biocidal material may be absorbed by ion exchange, using an aqueous solution containing a small concentration of silver ions, preferably less than 1.0 mM, but preferably not less than 0.01 mM. Alternatively, in principle, silver cations might be incorporated into the hydroxyapatite before it is used to coat the implant, for example by contact with a soluble silver salt, or by co-precipitation at the desired doping level; however the plasma spraying step may decrease the degree to which silver can leach out from the final coating.

With an implant of a titanium-based alloy, the surface may also be treated to absorb silver ions by anodising substantially the entire surface of the implant structure in, for example, phosphoric acid at a voltage above 50 V for a period of at least 30 minutes, at a current no greater than 20 mA/cm$^2$, so as to generate a surface oxide layer in which there are small pits of ion absorbent material. Silver ions can then be incorporated, primarily in the material in these pits, to provide a biocidal effect. Although this anodising step is not the normal way of treating a surface prior to such deposition of hydroxyapatite, surprisingly the hydroxyapatite has been found to adhere very well to this very hard oxide surface with small pits. Possibly more surprisingly the coating of hydroxyapatite does not inhibit leaching of silver ions from the anodised surface, and so providing a biocidal effect when the implant is in a human or animal body.

Performing the anodising at a voltage above 50 V and with a current limitation has two effects: it generates a dense hard surface layer whose thickness is primarily determined by the voltage, and it then generates shallow pits in the surface which are filled with a somewhat softer and more porous material. The concentration of phosphoric acid is preferably at least 1 M, more preferably between 2 and 3 M, with the preferred anodising voltages. The subsequent adsorption of biocidal metal ions is primarily into the material within the shallow pits, so that the total quantity of biocidal material can be controlled by controlling the magnitude of the anodising voltage and its duration, so as to control the number and size of the shallow pits.

The anodising might be carried out at a voltage as high as 500 V or 750 V, but preferably is performed between 50 V and 150 V. The duration may be up to 24 hours, but preferably no more than 12 hours, for example 0.5 hours, 2 hours or 6 hours. One benefit of performing the anodising at a voltage in this range is that the surface finish is not deleteriously affected; if part of the surface is polished before anodising so as to be shiny, then it will remain shiny after the high-voltage anodising step. This is in contrast to the effect of low voltage anodising, which makes the surface look milky or matt.

The metal structures of prosthetic implants are typically of a form of stainless steel, a titanium alloy, or a cobalt/chromium alloy. The standard alloys for this purpose are titanium 90% with 6% aluminium and 4% vanadium (British standard 7252), or chromium 26.5-30%, molybdenum 4.5-7%, and the remainder cobalt (British standard 7252 part 4) although this invention is not restricted to such examples. The metal structures of such prosthetic implants can also be metals including niobium, tantalum and zirconium and alloys thereof. The provision of a hydroxyapatite coating containing biocidal ions is applicable to such metal structures, of whatever material they are made.

The invention will now be further and more particularly described, by way of example only, and with reference to the accompanying drawings in which:

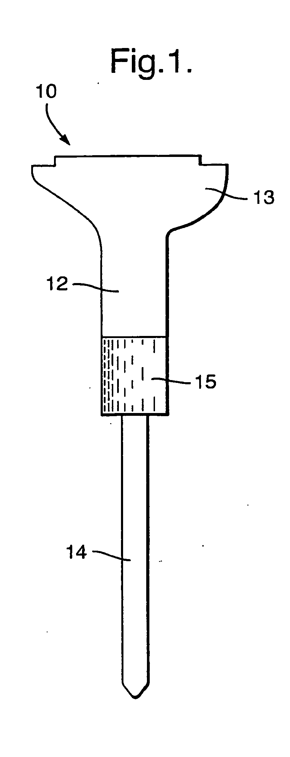

Figure 1 shows a side view of an implant for use as a proximal tibia prosthesis.

An implant for use as a proximal tibia prosthesis comprises a structure 10 made of titanium alloy (Ti/A1/V). It consists of three parts: an upper part 12 which is to replace the proximal part of a tibia, broadening out at its upper end 13 to form the under part of a knee joint; and a lower part 14 of narrower diameter to locate within a corresponding hole in the remaining part of the tibia. It will be appreciated that the implant structure 10 is of dimensions that are specific for use with a particular patient. A lower section 15 of the upper part 13 will, when implanted, be in contact with bone, and it is therefore desirable that bone should bond to the surface of the section 15.

The implant structure 10 is preferably polished mechanically or by using an electropolishing technique, so that all the surfaces are shiny. The surface has a pale grey colour. The surface of the lower section 15 is then subjected to grit-blasting with alumina powder, the remaining surfaces being masked using standard grit-blasting masking to protect the highly polished surface. The grit-blasting is such as to achieve a surface roughness of about Rz = 4.2 $\mu$m. The masking is then removed. The implant structure 10 is cleaned ultrasonically using first acetone as the liquid phase, and then a 1 M aqueous solution of sodium hydroxide, and is then rinsed in de-ionised water.

The cleaned implant structure 10 is then immersed in a stirred solution of phosphoric acid of between 1 M and 5 M, for example 2.1 M, and is anodised for 2 hours at a maximum voltage of 100 V and a maximum current of 10 mA/cm$^2$, so as to form a surface coating of titanium oxide and phosphate. Initially the current would tend to be significantly greater than this, so the current is limited; after a couple of minutes the current decreases to below this limit as a dense dielectric layer is formed on the surface, and the current then adopts a stable low value for the rest of the anodising period. The surface forms a hard surface oxide layer which can have different coloured appearances due to optical interference effects; during the initial stage of anodising, the surface colour varies from purple/blue, through blue, green, yellow, orange, and then finally red. Anodising at 100 V produces a film thickness of about 0.14 $\mu$m (140 nm). The anodised implant structure 10 is then rinsed in deionised water again.

The implant structure 10 is then immersed in a stirred 0.1 M aqueous solution of silver nitrate, and left for 2 hours. As a result of ion exchange there is consequently some silver adsorbed into the surface coating. The effect of the high voltage and low current anodising, in this phosphoric acid electrolyte, is that the surface forms a hard anodised oxide layer typically of thickness about 0.14 $\mu$m, but in which there are pits typically of diameter about 5 $\mu$m and depth about 0.4 $\mu$m which are filled with titanium oxide as a result of hydrolysis from localised titanium dissolution. Such pits are approximately circular in plan, and make up between 15 and 20% of the surface area. Surface analysis techniques have confirmed that, after ion exchange treatment, the adsorbed silver is associated with the titanium oxide/phosphate phase at the surface. Silver is absorbed to a small extent at the outer surface of the hard layer, and to a larger extent within the more porous material in the pits.

Thus the effects of anodising at 100 V for 2 hours are to produce a hard and compact oxide layer whose thickness depends upon the voltage (the relationship being approximately 1.4 nm per volt), this film having a coloured appearance determined by the film thickness, and retaining the surface microstructure (polished finish in parts, and rough finish in other parts). The surface is pitted on a microscopic scale, this not affecting the appearance. The anodised surfaces can be loaded with silver in range 0.1 to 20 $\mu$g/cm$^2$ and typically at about 5 to 9 $\mu$g/cm$^2$.

The implant structure 10 is then masked on all the shiny surfaces with woven glass-fibre heat-resistant tape up to about 50 mm of the edge of the shiny section, and a 100 mm wide strip of silver foil is then used to mask up to the edge of the shiny section, part of this foil overlying the glass-fibre tape. This is then covered with nickel foil (to reduce the risk of damage during handling). The rough surface (that of the section 15) is then coated with hydroxyapatite by plasma spray coating, to a thickness of about 80 $\mu$m. The foil ensures that hydroxyapatite is not deposited onto the shiny surfaces, and also ensures that the ultraviolet radiation from the plasma does not irradiate the shiny surfaces, which might reduce the adsorbed is ions to metal; this is not an issue with the rough surfaces, as they are shielded by the deposited hydroxyapatite itself.

The masking is then removed, and implant is given a final ultrasonic clean using isopropyl alcohol as a solvent. It is then ready for use in a patient. Surprisingly the hydroxyapatite has been found to adhere well to the hard oxide layer; and the hydroxyapatite coating on the rough surface does not prevent silver ions from being gradually leached out from the anodised layer into the surrounding body fluids, after implantation, so that any bacteria in the immediate vicinity of the implant are killed. Infection arising from the implant is therefore suppressed. And the coating of hydroxyapatite enhances bone growth onto the implant. The hydroxyapatite is a white coating.

After deposition of a hydroxyapatite coating, the hydroxyapatite coating is immersed in a dilute solution of a silver salt, for example 0.33 mM (0.00033 M) aqueous silver nitrate. Preferably this solution is made up using de-ionised water. At such low concentrations of silver ions there is a limited degree of ion exchange with hydroxyapatite, with formation of $Ag_2HPO_4$, which is white. By way of example, the loading of silver in the hydroxyapatite coating after 2 hours immersion at 20°C has been found to be 5.9 $\mu$g/cm$^2$. The silver loading increases with the concentration of silver in the solution, and for example with a 0.5 mM silver nitrate solution the silver loading after 2 hours immersion at 20°C was about 22.9 $\mu$g/cm$^2$; while with a 1.0 mM silver nitrate solution the silver loading under the same conditions was about 48.4 $\mu$g/cm$^2$. If the desired loading is between 5 and 10 $\mu$g/cm$^2$, this may be achieved using a solution of between about 0.3 mM and 0.4 mM. Experimentally it has been found that the silver loading, P (in $\mu$g/cm$^2$) is related to the silver concentration in the solution, C (molarity), by the equation:

$$\log P = (1.334 \times \log C) + 5.5$$

where the logarithms are to base 10.

The amount of silver absorbed is not significantly affected by the temperature (at least for temperatures in the ambient range), and is not significantly affected by the time of immersion, at least for an immersion of at least 0.5 hours. The 2 hour immersions described above have been found to lead to absorption of between about 5% and 15% of the silver ions in solution.

If the concentration of silver nitrate solution is as high as 1 mM there is a slight discoloration of the white hydroxyapatite surface. And if the hydroxyapatite coating is immersed in 10 mM silver nitrate solution under the same conditions, the surface goes pale yellow with formation of silver phosphate; the silver loading in this case was found to be about 555 $\mu$g/cm$^2$. This silver loading is higher than is required for satisfactory biocidal properties of the implant; the yellow coloration is unattractive; and there is a risk that the surface will become grey if exposed to light (due to photo-reduction of silver ions to silver).

It will be appreciated that the incorporation of silver ions into hydroxyapatite can be carried out as described above, whatever the metal of the structure may be. The hydroxyapatite may be coated onto anodised titanium (as described above), or onto non-anodised titanium, or cobalt chrome alloy, or any other suitable metal.

In a modification of the overall process as described above, a titanium metal implant is anodised to provide the surface with ion exchange properties; the roughened part of the surface is then coated with hydroxyapatite; and then ion exchange is carried out with both the hydroxyapatite-coated and the uncoated treated parts of the surface, using a sufficient concentration of silver ions to provide a loading above 2 $\mu$g/cm$^2$ in both the hydroxyapatite-coated and uncoated parts.

Providing silver loading in both a hydroxyapatite coating, preferably at a level no more than 30 $\mu$g/cm$^2$, and also into the surface of the metal implant, increases the silver that is available to leach out into the body fluids after implantation, and so enhances the biocidal properties of the implant.

After implantation into the body, silver ions gradually leach out of the hydroxyapatite coating into the adjacent body fluids, so ensuring a biocidal effect. The leach rate has been found to be controlled by the solubility of silver chloride. An experimental test has been carried out using a silver-loaded hydroxyapatite coating on a cobalt chrome alloy structure, this being immersed in 500 ml of 0.9% NaCl aqueous solution kept stirred and held at 35°C over a period of two weeks.

Each day a sample of 50 ml was withdrawn for analysis, and replaced with fresh NaCl solution (representing for example the gradual replacement of sinovial fluid around a joint). In this example the initial quantity of silver was about 60 $\mu$g/cm$^2$ (about twice the maximum desired level). Throughout the period of the experiment the measured concentration of silver in the solution remained between about 0.4 and 0.6 ppm; this is in conformity with the expected concentration based on the solubility of silver chloride, which would be 0.48 ppm (of silver). The time for which the biocidal effect would be operative, after implantation, is consequently controlled by the rate of loss of fluid from around the implant surgery site, along with the initial silver loading (which in the experiment described above decreased by about 16% over the two weeks).

## Claims

1. An implant suitable for use at least partly in contact with bone, the implant comprising a metal structure, wherein the surface of the metal structure has an anodised hard oxide surface in which are small pits of ion absorbent material, wherein there is a ceramic coating containing hydroxyapatite deposited onto the anodised oxide at at least part of the surface of the metal structure; wherein silver ions which can gradually leach out into body fluids after implantation are contained within the ceramic coating or the anodised surface layer or both.

2. An implant as claimed in claim 1 wherein the metal of the metal structure comprises titanium.

3. An implant as claimed in claim 1 or claim 2 wherein a part of the implant to be in contact with bone has a rough surface to the metal structure, the rough surface being provided with the ceramic coating.

4. An implant as claimed in any one of claims 1 to 3 wherein the loading of silver ions in the ceramic coating is between 0.1 and 30 $\mu$g/cm$^2$.

5. A method of making an implant suitable for use at least partly in contact with bone, the implant comprising a metal structure, the method comprising the steps of depositing onto at least part of the surface of the metal structure a ceramic coating containing hydroxyapatite by thermal spraying using a plasma spray system, and incorporating silver ions into the ceramic coating which can gradually leach out into body fluids after implantation.

6. A method as claimed in claim 5 wherein the metal of the metal structure is principally titanium, and the metal structure is treated by anodising to generate a hard oxide surface in which are small pits of ion absorbent material, before deposition of the ceramic coating.

7. A method as claimed in claim 5 also comprising subjecting at least part of the surface of the metal structure to a roughening treatment prior to deposition of the ceramic coating.

8. A method as claimed in claim 6 also comprising subjecting the anodised surface to ion exchange to absorb silver, before deposition of the ceramic coating.

9. A method as claimed in claim 6 or claim 8 also comprising subjecting at least part of the surface of the metal structure to a roughening treatment prior to the anodising treatment.

10. A method as claimed in any one of claims 5 to 9 wherein the loading of silver ions in the ceramic coating is between 0.1 and 30 $\mu$g/cm$^2$.

11. A method as claimed in any one of claims 5 to 10 wherein the silver ions are incorporated by ion exchange into the deposited ceramic coating, by immersing the coated implant in a solution containing the silver ions.

12. A method as claimed in claim 11 wherein the ion exchange with the ceramic coating is carried out using an aqueous solution containing a concentration of silver ions between 0.00001 M and 0.001 M.

**13.** A method as claimed in any one of claims 5 to 12 wherein, during the thermal spraying, parts of the surface of the metal structure that are not to be provided with the ceramic coating are first masked with a masking that incorporates a metal foil.

**14.** An implant made by a method as claimed in any one of claims 5 to 13.

## Patentansprüche

**1.** Ein Implantat, das zur Verwendung in mindestens teilweisem Kontakt mit Knochen geeignet ist, wobei das Implantat eine Metallstruktur umfasst, wobei die Oberfläche der Metallstruktur eine anodisierte harte Oxidoberfläche aufweist, in der sich kleine Vertiefungen eines ionenabsorbierenden Materials befinden, wobei eine keramische Beschichtung vorhanden ist, die Hydroxylapatit beinhaltet, das auf das anodisierte Oxid an mindestens einem Teil der Oberfläche der Metallstruktur abgeschieden wurde; wobei Silberionen, die nach der Implantation stufenweise in Körperflüssigkeiten ausgewaschen werden können, in der keramischen Beschichtung oder der anodisierten Oberflächenschicht oder in beiden enthalten sind.

**2.** Ein Implantat wie in Anspruch 1 beansprucht, wobei das Metall der Metallstruktur Titan umfasst.

**3.** Ein Implantat wie in Anspruch 1 oder Anspruch 2 beansprucht, wobei ein Teil des Implantats, das mit Knochen in Kontakt sein soll, eine raue Oberfläche an der Metallstruktur aufweist, wobei die raue Oberfläche mit der keramischen Beschichtung bereitgestellt wird.

**4.** Ein Implantat wie in einem der Ansprüche 1 bis 3 beansprucht, wobei die Beladung an Silberionen in der keramischen Beschichtung zwischen 0,1 und 30 $\mu$g/cm$^2$ beträgt.

**5.** Ein Verfahren zur Herstellung eines Implantats, das zur Verwendung in mindestens teilweisem Kontakt mit Knochen geeignet ist, wobei das Implantat eine Metallstruktur umfasst, wobei das Verfahren die folgenden Schritte umfasst: Abscheiden auf mindestens einem Teil der Oberfläche der Metallstruktur einer keramischen Beschichtung, die Hydroxylapatit beinhaltet, durch thermisches Spritzen unter Verwendung eines Plasmaspritzsystems, und Einarbeiten von Silberionen in die keramische Beschichtung, die nach der Implantation stufenweise in Körperflüssigkeiten ausgewaschen werden können.

**6.** Ein Verfahren wie in Anspruch 5 beansprucht, wobei das Metall der Metallstruktur prinzipiell Titan ist und die Metallstruktur durch Anodisierung behandelt wird, um eine harte Oxidoberfläche, in der sich kleine Vertiefungen eines ionenabsorbierenden Materials befinden, vor der Abscheidung der keramischen Beschichtung zu erzeugen.

**7.** Ein Verfahren wie in Anspruch 5 beansprucht, das ferner vor der Abscheidung der keramischen Beschichtung das Unterwerfen mindestens eines Teils der Oberfläche der Metallstruktur einer Aufraubehandlung umfasst.

**8.** Ein Verfahren wie in Anspruch 6 beansprucht, das ferner vor der Abscheidung der keramischen Beschichtung das Unterwerfen der anodisierten Oberfläche einem Ionenaustausch, um Silber zu absorbieren, umfasst.

**9.** Ein Verfahren wie in Anspruch 6 oder Anspruch 8 beansprucht, das ferner vor der Anodisierungsbehandlung das Unterwerfen mindestens eines Teils der Oberfläche der Metallstruktur einer Aufraubehandlung umfasst.

**10.** Ein Verfahren wie in einem der Ansprüche 5 bis 9 beansprucht, wobei die Beladung an Silberionen in der keramischen Beschichtung zwischen 0,1 und 30 $\mu$g/cm$^2$ beträgt.

**11.** Ein Verfahren wie in einem der Ansprüche 5 bis 10 beansprucht, wobei die Silberionen durch Ionenaustausch in die abgeschiedene keramische Beschichtung eingearbeitet werden, indem das beschichtete Implantat in eine die Silberionen enthaltende Lösung eingetaucht wird.

**12.** Ein Verfahren wie in Anspruch 11 beansprucht, wobei der Ionenaustausch mit der keramischen Beschichtung unter Verwendung einer wässrigen Lösung durchgeführt wird, die eine Konzentration von Silberionen zwischen 0,00001 M und 0,001 M beinhaltet.

**13.** Ein Verfahren wie in einem der Ansprüche 5 bis 12 beansprucht, wobei, während des thermischen Spritzens, Teile der Oberfläche der Metallstruktur, die mit der keramischen Beschichtung nicht versehen werden sollen, zunächst mit einer Maskierung maskiert werden, die eine Metallfolie einschließt.

**14.** Ein Implantat, das durch ein in einem der Ansprüche 5 bis 13 beanspruchten Verfahren hergestellt wurde.

## Revendications

**1.** Implant approprié pour une utilisation au moins partiellement en contact avec de l'os, l'implant comprenant une structure métallique, la surface de la structure métallique ayant une surface d'oxyde dur anodisé dans laquelle se trouvent de petites piqûres de matière absorbant les ions, un revêtement céramique contenant de l'hydroxyapatite ayant été déposé sur l'oxyde anodisé en au moins une partie de la surface de la structure métallique ; des ions argent qui peuvent progressivement passer par lixiviation dans des fluides corporels après implantation étant contenus à l'intérieur du revêtement céramique ou de la couche de surface anodisée ou des deux.

**2.** Implant selon la revendication 1, dans lequel le métal de la structure métallique comprend du titane.

**3.** Implant selon l'une des revendications 1 ou 2, dans lequel une partie de l'implant devant être en contact avec de l'os a une surface rugueuse sur la structure métallique, la surface rugueuse étant dotée du revêtement céramique.

**4.** Implant selon l'une quelconque des revendications 1 à 3, dans lequel la charge d'ions argent dans le revêtement céramique se situe entre 0,1 et 30 $\mu$g/cm$^2$.

**5.** Procédé de fabrication d'un implant approprié pour une utilisation au moins partiellement en contact avec de l'os, l'implant comprenant une structure métallique, le procédé comprenant les étapes consistant à déposer sur au moins une partie de la surface de la structure métallique un revêtement céramique contenant de l'hydroxyapatite par pulvérisation thermique à l'aide d'un système de pulvérisation au plasma, et à incorporer des ions argent dans le revêtement céramique, lesquels peuvent progressivement passer par lixiviation dans des fluides corporels après implantation.

**6.** Procédé selon la revendication 5, dans lequel le métal de la structure métallique est principalement du titane, et la structure métallique est traitée par anodisation pour générer une surface d'oxyde dur dans laquelle se trouvent de petites piqûres de matière absorbant les ions, avant dépôt du revêtement céramique.

**7.** Procédé selon la revendication 5, comprenant également le fait de soumettre au moins une partie de la surface de la structure métallique à un traitement de rugosification avant dépôt du revêtement céramique.

**8.** Procédé selon la revendication 6, comprenant également le fait de soumettre la surface anodisée à un échange d'ions pour absorber l'argent, avant dépôt du revêtement céramique.

**9.** Procédé selon l'une des revendications 6 ou 8, comprenant également le fait de soumettre au moins une partie de la surface de la structure métallique à un traitement de rugosification avant le traitement d'anodisation.

**10.** Procédé selon l'une quelconque des revendications 5 à 9, dans lequel la charge d'ions argent dans le revêtement céramique se situe entre 0,1 et 30 $\mu$g/cm$^2$.

**11.** Procédé selon l'une quelconque des revendications 5 à 10, dans lequel les ions argent sont incorporés par échange d'ions dans le revêtement céramique déposé, par immersion de l'implant revêtu dans une solution contenant les ions argent.

**12.** Procédé selon la revendication 11, dans lequel l'échange d'ions avec le revêtement céramique est effectué à l'aide d'une solution aqueuse contenant une concentration d'ions argent entre 0,00001 M et 0,001 M.

**13.** Procédé selon l'une quelconque des revendications 5 à 12, dans lequel, durant la pulvérisation thermique, des parties de la surface de la structure métallique qui ne doivent pas être dotées du revêtement céramique sont tout d'abord masquées par un masquage qui incorpore une feuille métallique.

**14.** Implant obtenu par un procédé tel que défini à l'une quelconque des revendications 5 à 13.

# Fig.1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4746532 A
- WO 2005087982 A
- WO 03089023 A
- WO 2006058906 A
- WO 0394774 A

**Non-patent literature cited in the description**

- **M. Shirkhanzadeh.** *Materials Letters,* 1995, vol. 24, 7-12